# FASCICULE DE BREVET EUROPEEN

(11) **EP 1 390 474 B1**
(45) Date de publication et mention de la délivrance du brevet: **21.02.2007**
(21) Numéro de dépôt: 02740789.9
(22) Date de dépôt: 10.05.2002
(51) Int. Cl.: C12N 5/06, C12N 5/10, A61K 35/14, A61P 37/02, A61P 29/00

(54) **PROCEDE D'OBTENTION DE LYMPHOCYTES TR1 REGULATEURS SPECIFIQUES D'ANTIGENE**
VERFAHREN ZUR GEWINNUNG ANTIGENSPEZIFISCHER TR1-REGUALTORISCHER LYMPHOZYTEN
METHOD FOR OBTAINING ANTIGEN-SPECIFIC TR1 REGULATORY LYMPHOCYTES

(30) Priorité: 11.05.2001 FR 0106231
(43) Date de publication de la demande: 25.02.2004
(73) Titulaire: INSTITUT NATIONAL DE LA SANTE ET DE LA RECHERCHE MEDICALE (INSERM), 75013 Paris (FR)
(72) Inventeur: GROUX, Hervé, F-06410 Biot (FR); COTTREZ, Françoise, F-06410 Biot (FR); WAKKACH, Abdelilah, F-06100 Nice (FR)
(74) Mandataire: Vialle-Presles, Marie José
(86) Numéro de dépôt international: PCT/FR2002/001586
(87) Numéro de publication internationale: WO 2002/092793

(56) Documents cités:
- WO-A-97/29183
- WO-A-97/42324
- VINGERHOETS J ET AL: "Superantigen activation of CD4+ and CD8+T cells from HIV-infected subjects: Role of costimulatory molecules and antigen-presenting cells (APC)." CLINICAL AND EXPERIMENTAL IMMUNOLOGY, vol. 111, no. 1, janvier 1998 (1998-01), pages 12-19, XP002194073 ISSN: 0009-9104
- GROUX HERVE ET AL: "A CD4+ T-cell subset inhibits antigen-specific T-cell responses and prevents colitis" NATURE, MACMILLAN JOURNALS LTD. LONDON, GB, vol. 389, no. 6652, 16 octobre 1997 (1997-10-16), pages 737-742, XP002164695 ISSN: 0028-0836 cité dans la demande

## Description

L'invention concerne l'obtention de lymphocytes T CD4⁺ régulateurs, utilisables dans le cadre de la prévention et du traitement de maladies auto-immunes et inflammatoires.

Les maladies auto-immunes sont dues à une dérégulation du système immunitaire, qui se traduit par une réponse immune indésirable d'un organisme vis-à-vis de ses propres antigènes.

On a tenté de manipuler les antigènes à l'origine de ces pathologies ou les cellules T agressives spécifiques de ces antigènes, mais les résultats obtenus ont souvent été très limités notamment par l'absence d'une connaissance de tous les antigènes impliqués dans la pathologie concernée. En effet, les auto-antigènes proprement dits, ou les antigènes responsables des désordres inflammatoires et auto-immuns ne sont pas toujours connus, ou sont différents d'un individu à l'autre. Les traitements utilisés actuellement dans ces maladies sont soit des traitements palliatifs (insuline dans le cas du diabète, anti-histaminique dans le cas des désordres allergiques) soit des traitements systémiques par des anti-inflammatoires (AINS) et/ou des immuno-suppresseurs (glucocorticoïdes, cyclosporine, anticorps...). Il y a donc clairement un besoin pour un traitement immunosuppresseur puissant mais limité à l'organe atteint ou plus précisément à la zone d'hyperactivité du système immunitaire.

Parmi les nombreux agents impliqués dans la régulation de la réponse immune, on citera les lymphocytes T CD4⁺, également dénommés lymphocytes T auxiliaires (T *helpers* ). On distingue classiquement 2 types principaux de lymphocytes T auxiliaires : les lymphocytes Th1, impliqués dans le développement de la réponse immune cellulaire, produisent des cytokines pro-inflammatoires telles que l'interleukine-2 (IL-2) et l'interféron γ (IFNγ) et présentent des effets activateurs des macrophages ; les lymphocytes Th2, qui produisent des cytokines telles que les interleukines IL-4, IL-6, IL-10 et IL-13, favorisent la sécrétion d'anticorps.

Des travaux récents auxquels a participé l'un des Inventeurs ont abouti à la découverte d'une nouvelle catégorie de cellules T régulatrices, dont la prolifération est induite par l'activation de cellules T CD4⁺ en présence d'interleukine 10 (IL-10), et qui ont été dénommées lymphocytes Tr1 (Demande PCT WO 97/42324). La poursuite de ces travaux a permis l'isolement et la caractérisation d'une nouvelle sous-population de cellules T régulatrices, qui ont été dénommées lymphocytes Tr1 (GROUX et al., Nature, 389, 737-742; 1997). Cette sous-population lymphocytaire a été obtenue par activations répétées de cellules T CD4⁺ en présence d'un antigène et d'interleukine 10 (IL-10). Lorsqu'elles sont re-stimulées par l'antigène utilisé pour leur induction, les cellules Tr1 ne prolifèrent que faiblement, produisent des quantités très élevées d'IL-10, des quantités importantes de TGF-β (tumor growth factor β), de très faibles quantités d'IL-2, et ne produisent pas d'IL-4. Lorsque des cellules Tr1 activées sont cultivées en présence d'autres cellules T CD4⁺ elles suppriment la prolifération de celles-ci en réponse à un antigène ; cet effet résulte de la sécrétion de cytokines, et notamment d'IL-10, par les lymphocytes Tr, et non d'une action directe de ceux-ci sur les cellules T CD4⁺ ; il peut donc être obtenu sans avoir à connaître l'antigène responsable de la prolifération de ces cellules. Ceci présente un avantage important dans le cas de maladies auto-immunes, dont on peut ainsi envisager le traitement sans qu'il soit nécessaire de connaître l'antigène exact contre lequel sont dirigées les cellules pathogéniques.

Il a ainsi été observé, dans un modèle expérimental de maladie de Crohn chez la souris dans lequel les cellules pro-inflammatoires sont dirigées contre des bactéries commensales de la flore digestive, que l'administration aux animaux des cellules Tr1 dirigées contre l'ovalbumine, accompagnée de l'administration d'ovalbumine dans la nourriture, permettait de prévenir l'instauration d'une inflammation chronique du colon.

D'autre part, dans des travaux récents sur différents modèles animaux de maladie de Crohn, de sclérose en plaques, ou de réaction du greffon contre l'hôte, les Inventeurs ont montré que les cellules inhibitrices Tr1 étaient capables non seulement de prévenir, mais aussi de guérir ces différentes pathologies. Ils ont en outre observé que les cellules Tr1 migraient de façon spécifique et intense dans les différents sites inflammatoires. Cette propriété des cellules Tr1 représente un avantage supplémentaire dans l'utilisation de ces cellules comme vecteur de molécules anti-inflammatoires dans les sites d'intense inflammation. Des cellules Tr1 obtenues à partir des cellules T d'un patient sont donc potentiellement utilisables dans le cadre d'une thérapie cellulaire pour réguler la réponse immunitaire chez ce patient. Elles sont ainsi utilisables notamment pour prévenir ou soigner, non seulement les maladies auto-immunes et inflammatoires mentionnées ci-dessus, mais également toute autre pathologie caractérisée par une réponse inflammatoire aberrante, comme le diabète, le psoriasis, l'athérosclérose, la polyarthrite rhumatoïde ou l'asthme ; elles sont également utilisables dans le traitement des rejets de greffe ou de la réaction du greffon contre l'hôte.

La méthode d'obtention des lymphocytes Tr1 qui est décrite dans la publication de GROUX *et al*. citée ci-dessus, nécessite la stimulation répétée des cellules T avec l'antigène en présence d'IL-10. Cette méthode est lourde à mettre en oeuvre, et ne permet pas d'obtenir rapidement, à partir d'un patient, une population de lymphocytes Tr1 utilisable dans le cadre d'un traitement thérapeutique.

Différentes équipes ont rapporté l'implication de différentes molécules de co-stimulation dans l'obtention de cellules possédant des caractéristiques similaires à celles des cellules Tr1, notamment au niveau de la production d'IL-10 ; les molécules de stimulation potentiellement impliquées sont très diverses, et les effets rapportés apparaissent parfois contradictoires.

BLEIJS *et al.* (Eur. J. Immunol., 29, 2248, 1999) ont ainsi observé que la co-stimulation par l'interaction de LFA-1 avec ICAM-1, ICAM-2 ou ICAM-3 induit une production importante de GM-CSF (Granulocyte-macrophage colony-stimulating factor), d'IFN-γ, et d'IL-10 par des lymphocytes T stimulés par un anticorps anti-CD3. La production d'IL-10 est particulièrement importante lors de la co-stimulation par l'interaction LFA-1/ICAM-1. BULLENS *et al*. (Int. Immunol. 13, 181-191, 2001) rapportent que la co-stimulation par CD58 induit la production d'IL-10 et d'IFN-γ par des cellules T stimulées par un anticorps anti-CD3. DONG *et al*. (Nat. Med., 5, 1365, 1999) ont décrit une molécule de co-stimulation apparentée à la famille B-7, et dénommée B7-H1, qui induit préférentiellement la sécrétion d'IL-10.

VAN GOOL *et al.* (Eur. J. Immunol., 29, 2367, 1999) ont observé que l'activation de cellules T par un alloantigène en présence d'anticorps bloquant les interactions CD80/CD28 ou CD86/CD28 et CD40/CD40L induisait une anergie spécifique d'antigène, accompagnée d'une diminution de la production d'IFN-γ et d'une augmentation de la production d'IL-10.

CHABOT *et al*. (J. Immunol., 162, 6819, 1999) rapportent que la production d'IL-10, induite par l'interaction de cellules microgliales avec des lymphocytes T est diminuée par le blocage de CD40/CD40L, B7/CTLA-4 ou B7/CD28, ou de CD23.

JONULEIT et al. (J. Exp. Med. 192, 1112, 2000) décrit la production des cellules T régulatrices à partir des cellules dendritiques immatures (CD83⁻).

Les Inventeurs ont maintenant mis au point un nouveau procédé permettant d'obtenir rapidement des lymphocytes Tr1 antigène-spécifiques, en quantité bien supérieure à celle obtenue par l'activation de cellules T CD4+ en présence d'IL-10. Ils ont en effet constaté que la culture de cellules T CD4+ en présence d'un antigène inducteur, et de cellules présentatrices de l'antigène exprimant une molécule HLA de classe II et la molécule LFA-3 (CD58) humaine, mais n'exprimant aucune des molécules co-stimulatrices B7-1 (CD80), B7-2 (CD86), B7-H1, CD40, CD23 et ICAM-1 (CD54), induisait la différenciation de cellules Tr1 spécifiques dudit antigène.

La molécule LFA-3 est le ligand du récepteur CD2 des lymphocytes T. Deux formes de LFA 3 ont été décrites : une forme transmembranaire (WALNER *et al.,* J. Exp. Med. 166, 923-932, 1987 ; Demande PCT WO 88/09826) et une forme, dénommée " PI-linked LFA3 " qui est ancrée à la membrane cellulaire par l'intermédiaire d'un glycolipide contenant du phosphatidylinositol (Demande PCT WO 90/02181).

La présente invention a pour objet l'utilisation de cellules présentatrices de l'antigène artificielles, exprimant une molécule du système HLA de classe II et une molécule LFA-3 humaine, et n'exprimant aucune des molécules de co-stimulation B7-1, B7-2, B7-H1, CD40, CD23 ou ICAM-1 (CD54), pour l'obtention de lymphocytes régulateurs Tr1 spécifiques d'antigène.

On définit comme lymphocytes régulateurs Tr1 spécifiques d'antigène, des cellules possédant les caractéristiques suivantes, après re-stimulation par ledit antigène :
- elles produisent une quantité élevée d'IL-10, à savoir une quantité égale ou supérieure à 3 x 10³ pg/10⁶ cellules, et généralement de 5 x 10³ à 20 x 10³ pg d'IL-10 pour 10⁶cellules ;
- elles produisent une quantité d'IL-2 égale ou inférieure à 50 pg/10⁶ cellules ; le rapport IL-10/IL-2 produites par lesdites cellules est au moins égal à 50/1, généralement de 100/1 à 500/1 ;
- elles produisent une quantité d'IL-4 égale ou inférieure à 50 pg/10⁶ cellules ; le rapport IL-10/IL-4 produites par lesdites cellules est au moins égal à 300/1, généralement de 500/1 à 10000/1;
- la prolifération des cellules TCD4+ stimulées par un antigène en présence de cellules Tr1 est diminuée d'au moins 2 fois, généralement d'au moins 2 à 100 fois.

La présente invention a notamment pour objet un procédé de préparation de lymphocytes régulateurs Tr1. spécifiques d'antigène à partir des lymphocytes d'un patient, caractérisé en ce qu'il comprend :
- l'activation *in vitro* desdits lymphocytes en présence de l'antigène choisi présenté par des cellules présentatrices de l'antigène artificielles telles que définies ci-dessus ;
- la récupération, à partir desdits lymphocytes, d'une population de lymphocytes T CD4+ activés comprenant au moins 10%, de préférence au moins 50%, et de manière tout à fait préférée, au moins 80% de lymphocytes Tr1 spécifiques dudit antigène.

Des cellules présentatrices de l'antigène artificielles utilisables pour la mise en oeuvre de la présente invention, peuvent avantageusement être obtenues par co-transfection de cellules animales n'exprimant aucune des molécules de co-stimulation indiquées ci-dessus avec une séquence d'acide nucléique codant pour la chaîne α d'une molécule HLA de classe II, une séquence d'acide nucléique codant pour la chaîne β d'une molécule HLA de classe II et une séquence d'acide nucléique codant l'une quelconque des 2 formes du LFA-3. Si on le souhaite, ces cellules peuvent être également transfectées avec une séquence d'acide nucléique codant pour l'antigène vis-à-vis duquel on souhaite induire la spécificité des lymphocytes Tr1. Ces séquences d'acide nucléique peuvent être portées par des molécules d'acide nucléique différentes, ou bien 2 ou plus d'entre elles peuvent être portées par la même molécule d'acide nucléique.

Des cellules animales pouvant être utilisées pour l'obtention desdites cellules présentatrices de l'antigène artificielles peuvent être des cellules d'origine humaine, autologues ou hétérologues, ou bien des cellules d'origine xénogénique, notamment des cellules de mammifère. On peut utiliser des cultures primaires récemment isolées ; généralement on préférera utiliser des lignées cellulaires établies, qui sont plus homogènes, et capables de proliférer pendant plusieurs générations.

Il peut s'agir par exemple de fibroblastes, de kératinocytes, de cellules de tubules de rein, de cellules de Schwann, de myoblastes, de cellules endothéliales, etc.

La molécule de CMH de classe II exprimée par les cellules présentatrices de l'antigène artificielles sera choisie parmi les différentes molécules HLA-2 humaines disponibles, en fonction du type HLA-classe II du patient à partir duquel sont prélevés de lymphocytes T CD4+, et de l'utilisation envisagée pour les cellules Tr1.

On utilisera généralement des cellules présentatrices de l'antigène artificielles exprimant une molécule HLA-2 également exprimée par le patient ; toutefois, on peut également choisir une molécule HLA-2 différente de celles exprimées par ledit patient ; il peut s'agir par exemple; si l'on souhaite produire des cellules Tr1 utilisables dans le cadre de la prévention du rejet de greffe, d'une molécule HLA-2 exprimée par les cellules du greffon ; dans ce cas la molécule HLA-2 constitue l'antigène vis-à-vis duquel seront dirigées les cellules Tr1 obtenues.

L'antigène utilisé pour la mise en oeuvre du procédé conforme à l'invention sera choisi en fonction de l'utilisation à laquelle sont destinées les cellules Tr1 spécifiques d'antigène que l'on souhaite produire. Il peut s'agir d'un antigène associé à une pathologie inflammatoire ou auto-immune ; il peut s'agir également d'un antigène sans rapport avec une pathologie particulière, mais qui pourra être administré si l'on souhaite activer les cellules Tr1 pour lutter contre une réponse immune indésirable.

Le chargement de l'antigène sur les cellules présentatrices de l'antigène artificielles peut s'effectuer de manière classique, par co-incubation des cellules et dudit antigène. Celui-ci peut se présenter sous forme native, et être apprêté par lesdites cellules présentatrices de l'antigène ; il peut également se présenter sous forme d'un ou plusieurs peptides antigéniques, pouvant être fixés directement par les molécules HLA-2 exprimées par lesdites cellules. Alternativement, l'antigène peut être exprimé par les cellules présentatrices de l'antigène artificielles, dans le cas où celles-ci ont été préalablement transfectées avec une séquence d'acide nucléique codant pour celui-ci.

Pour la mise en oeuvre du procédé conforme à l'invention, l'activation *in vitro* des lymphocytes peut s'effectuer directement sur des PBMCs (cellules mononucléées du sang périphérique) prélevées à partir dudit patient. Elle peut également s'effectuer sur des lymphocytes T CD4+ préalablement purifiés à partir de ces PBMCs.

L'activation des lymphocytes s'effectue par co-culture de ceux-ci, pendant 2 à 10 jours, de préférence pendant 6 à 8 jours, en présence des cellules présentatrices de l'antigène artificielles décrites ci-dessus, chargées avec l'antigène choisi.

A l'issue de cette période de culture, on sélectionne les lymphocytes T CD4+ activés sur la base de l'expression du marqueur CD4+, et d'un ou plusieurs marqueurs d'activation tels que CD25, CD69, CD45RO, etc...

A l'issue de cette période de culture, on obtient une population cellulaire dans laquelle les lymphocytes Tr1 représentent au moins 10% et généralement entre 50 et 80% des lymphocytes T antigène-spécifiques activées.

Pour enrichir encore cette population en lymphocytes Tr1, on peut répéter la stimulation dans les conditions définies ci-dessus.

On obtient alors une population de lymphocytes T CD4+ activés comprenant au moins 30% et généralement entre 60 et 90% de lymphocytes Tr1 parmi les cellules T antigène spécifiques.

On peut également isoler des clones de lymphocytes Tr1 à partir de cette population. Ces clones peuvent aisément être identifiés sur la base du profil caractéristique de production de cytokines des lymphocytes Tr1, tel que défini ci-dessus.

L'expansion des clones peut être effectuée dans un milieu nutritif contenant des facteurs non-spécifiques de croissance tels que IL-4 et IL-2. Une réalisation préférentielle est d'utiliser comme agent de stimulation des billes couplées avec des anticorps anti-CD3 et CD28.

Les cellules Tr1 obtenues par le procédé conforme à l'invention présentent toutes les caractéristiques des cellules Tr1 obtenues par activation de lymphocytes T CD4+ en présence d'IL-10, et ont donc les mêmes utilisations que celles-ci pour la régulation de la réponse immunitaire.

La présente invention a également pour objet un procédé de préparation d'une composition pharmaceutique destinée au traitement de maladies inflammatoires et/ou de maladies autoimmunes, caractérisé en ce qu'il comprend la préparation de lymphocytes Tr1 spécifiques d'antigène par un procédé conforme à l'invention, et le conditionnement desdits lymphocytes Tr1 sous une formulation appropriée à leur administration à un patient.

La présente invention sera mieux comprise à l'aide du complément de description qui va suivre, qui se réfère à des exemples non-limitatifs illustrant la mise en oeuvre du procédé conforme à l'invention.

### EXEMPLE 1 : PREPARATION DE LYMPHOCYTES Tr

### Isolement de cellules T CD4⁺

Des cellules mononucléées périphériques (PBMC) sont obtenues par centrifugation sur Ficoll-Hypaque.

Les cellules T CD4⁺ sont obtenues par élimination des cellules non-CD4⁺, en utilisant des anticorps anti-CD8 (L533), anti-CD11b (OKM1), et anti-CD20(2H7), selon le protocole suivant. Les cellules sont incubées avec des concentrations saturantes d'anticorps pendant 20 minutes à 4°C. Après lavage, on ajoute des billes DYNABEADS (DYNAL, Oslo, Norvège), à raison d'un rapport billes/cellules-cibles de 1/1, et l'on incube pendant 1 heure à 4°C. Les billes portant les cellules non-CD4⁺ sont éliminées par application d'un champ magnétique. L'analyse par cytofluorométrie de flux (FACSstar, BECTON DICKINSON) des cellules restantes montre qu'elles comprennent 90 à 95% de cellules T CD4⁺.

### Obtention de cellules présentatrices de l'antigène artificielles

Des cellules fibroblastiques L (ATCC CCL-1) de souris ont été co-transfectées avec des séquences d'acide nucléique codant pour LFA-3 et pour une molécule HLA de classe II.

### Obtention d'un ADNc codant pour LFA-3

Un ADNc codant pour LFA-3 a été préparé à partir d'une banque d'ADNc total de cellules mononucléées périphériques humaines, par amplification en chaîne par polymérase (PCR) en utilisant l'amorce dénommée 5'LFA-3, qui comprend la séquence des nucléotides 13 à 33 de la séquence codant pour le LFA-3 humain (numéro d'accès GENBANK X06296), flanquée en (5' ) d' un site KpnI, et l'amorce dénommée 3'LFA-3, qui comprend la séquence des nucléotides 708 à 728 de la séquence codant pour le LFA-3 humain, flanquée en (5') d'un site NotI.

### Obtention d'ADNc codant pour DR1

Un ADNc codant pour la chaîne alpha de DR1 a été obtenu à partir d'une banque d'ADNc total de cellules mononucléées périphériques humaines d'un donneur DR1+, par amplification en chaîne par polymérase (PCR) en utilisant les amorces dénommées DR1A5' et DR1A3', qui comprennent respectivement la séquence des nucléotides 151 à 176, et la séquence des nucléotides 769 à 792 de la séquence codant pour la chaîne alpha de DR1 (numéro d'accès GENBANK KO1171). Un ADNc codant pour la chaîne bêta de DR1 a été obtenu de la même manière en utilisant les amorces dénommées DR1B5' et DR1B3' qui comprennent respectivement la séquence des nucléotides 52 à 74 et 850 à 923 de la séquence codant pour la chaîne bêta de DR1 (numéro d'accès GENBANK NMO02124).

### Transfection des cellules

Chacun des ADNc obtenus a été cloné dans un vecteur pcDNA 3.1/hygro (INVITROGEN), au site TA. Les vecteurs obtenus ont été utilisés pour co-transfecter les cellules L par électroporation.

Les transfectants stables sont triés par cytofluorométrie de flux (FACSVantage SE, BECTON DICKINSON) en marquant les cellules à l'aide d'un anticorps anti-LFA-3 (1C3) et d'un anticorps anti-DR (L243). Les cellules exprimant à la fois LFA-3 et DR sont retenues, et mises en culture sur milieu F12 (LIFE TECHNOLOGIES) supplémenté avec 10% de sérum de veau foetal (BOEHRINGER), et additionné de pénicilline et de streptomycine.

Des cellules présentatrices de l'antigène co-exprimant LFA-3 et DR1 ont également été préparées, comme décrit ci-dessus, par co-transfection de cellules P815 (ATCC-TIB64).

A titre de témoins, des cellules L ou P815 non-transfectées, ou des cellules obtenues par transfection de cellules L ou P815, et exprimant uniquement la molécule DR1 ont également été utilisées.

### Obtention de lymphocytes Tr

Des cellules T CD4⁺ isolées comme décrit ci-dessus, à partir de PBMCs d'un donneur non-DR1 sont suspendues, à raison de 2 x 10⁶ cellules/ml, dans du milieu de YSSEL (YSSEL *et al.,* J. Immunol. Methods, 72, 219-227, 1984).

La suspension cellulaire est répartie dans une plaque de culture à 24 puits, à raison de 1 ml par puits. Des cellules transfectées L-DR1-LFA3 ou des cellules transfectées L-DR1, obtenues comme décrit ci-dessus, ou à titre de témoin, des cellules lymphoblastoides B-EBV DR1+ sont irradiées (60 Gy) et ajoutées dans chaque puits, à raison de 5 x 10⁵ cellules/ml.

Après incubation pendant 7 jours, les cellules sont récoltées et lavées dans du PBS, puis marquées à l'aide d'anticorps anti-CD4 (RPA-T4) et anti-CD25 (M-A251). Les cellules CD4⁺CD25⁺ sont triées par cytofluorométrie de flux, et sont clonées à raison de 1 cellule/puits dans une plaque à 96 puits, dans du milieu de YSSEL. L'expansion clonale est effectuée en présence d'IL-2 et d'IL-4 par la technique décrite par SPITS et YSSEL (J. Immunol. Methods, 9, 416-421, 1996). Après expansion clonale, les différents clones sont re-stimulés par des cellules lymphoblastoides B-EBV DR1+. 48 heures après stimulation, le surnageant cellulaire est récupéré et le profil de production des cytokines IL-10, et IFN-γ en réponse à cette stimulation a été déterminé par dosage de ces cytokines par ELISA selon le protocole décrit par ABRAMS et al.( Curr. Protocols Immunol., 13, pp 6.1-6-15, 1995).

Les résultats illustrés dans le Tableau I ci-dessous représentent la production de cytokines IL-10, et IFN-γ de cellules Tr1 (moyenne ± écart type de 10 clones) de 2 donneurs différents.

**Tableau 1**

| Cellule | Donneur 1 | | Donneur 2 | |
|---|---|---|---|---|
| | IL-10(pg/ml) | IFN-γ(pg/ml) | IL-10(pg/ml) | IFN-γ(pg/ml) |
| L-DR1 | 326±40 | 13372±1500 | 3657±324 | 9638±63 |
| L-DR1-LFA-3 | 2315±183 | 19403±975 | 6402+272 | 914±322 |
| B-EBV DR1+ | <40 | 16005±123 | 174±59 | 4930±685 |

Dans une autre série d'expérimentations, les cellules T CD4⁺ isolées à partir de PBMCs d'un donneur DR1⁺ sont mélangées, comme décrit ci-dessus, avec des cellules présentatrices de l'antigène exprimant la molécule DR1 et la molécule LFA-3.

Un antigène inducteur (peptide HA 307-319, correspondant à un fragment de l'antigène de capside du virus *Haemophilus influenzae*) est ajouté au mélange de cellules, à raison de 50 µg/ml.

Après incubation pendant 3 jours, les cellules CD4⁺CD25⁺ sont triées par cytofluorométrie de flux, et sont clonées comme décrit ci-dessus.

Après expansion clonale, les différents clones sont re-stimulés par des cellules lymphoblastoides B-EBV chargées avec le peptide HA (10 µM). 48 heures après stimulation, le surnageant cellulaire est récupéré et le profil de production de cytokines IL-2, IL-4, IL-10, et IFN-γ en réponse à cette stimulation a été déterminé par dosage de ces cytokines par ELISA selon le protocole décrit par ABRAMS et collaborateurs.

La majeure partie (environ 70%) des clones CD4⁺CD25⁺ isolés présentent le profil de production de cytokines des cellules Tr1.

Les profils de production de cytokines de 9 de ces clones Tr1 sont illustrés par le tableau II ci-dessous.

**Tableau II**

| Clone | IL-2 (pg/ml) | IL-4 (pg/ml) | IL-10 (pg/ml) | IFN-γ (pg/ml) |
|---|---|---|---|---|
| HA-1A12 | <20 | <40 | 12358 | 521 |
| HA-1B6 | <20 | <40 | 11897 | 497 |
| HA-1C9 | <20 | <40 | 14598 | 1369 |
| HA-1E5 | <20 | <40 | 13549 | 314 |
| HA-1E7 | 40 | <40 | 11697 | 876 |
| HA-1F2 | <20 | <40 | 10597 | 1057 |
| HA-2B6 | <20 | <40 | 17891 | 697 |
| HA-2D5 | 32 | <40 | 16589 | 503 |
| HA-2F2 | <20 | <40 | 17803 | 873 |

Des clones de cellules Tr1 possédant le même profil de production de cytokines ont également été obtenus en utilisant comme cellules présentatrices de l'antigène les cellules P815-DR1-LFA-3, ce qui montre que l'obtention de cellules Tr1 n'est pas liée aux propriétés de la lignée cellulaire à partir de laquelle sont obtenues les cellules présentatrices de l'antigène.

### EXEMPLE 2 : PROPRIETES IMMUNOREGULATRICES DES LYMPHOCYTES TR1 OBTENUS PAR LE PROCEDE CONFORME A L'INVENTION

Les propriétés immunorégulatrices des cellules Tr1 antigène spécifique ont été testées comme suit :

Des cellules T CD4+ humaines issues d'un donneur DR1+ (1 x 10⁶/ml) sont stimulées par des monocytes syngéniques (1 x 10⁶/ml) irradiés (60 GY), en présence du peptide HA 307-319 (50 µM) et d'anatoxine tétanique (TT :50 µg/ml).

Parallèlement, la même expérimentation est effectuée en ajoutant des cellules (2 x 10⁵/ml) d'un clone Tr1 HA spécifique obtenu comme décrit ci-dessus, seules ou en présence d'un anticorps dirigé contre le récepteur d'IL-10 (anti-IL-10R, 10 µg/ml), d'un anticorps anti-TGF-β(20 µg/ml), ou d'un mélange de ces 2 anticorps.

La prolifération des cellules T CD4+ est déterminée après 5 jours, par mesure de l'incorporation de thymidine tritiée.

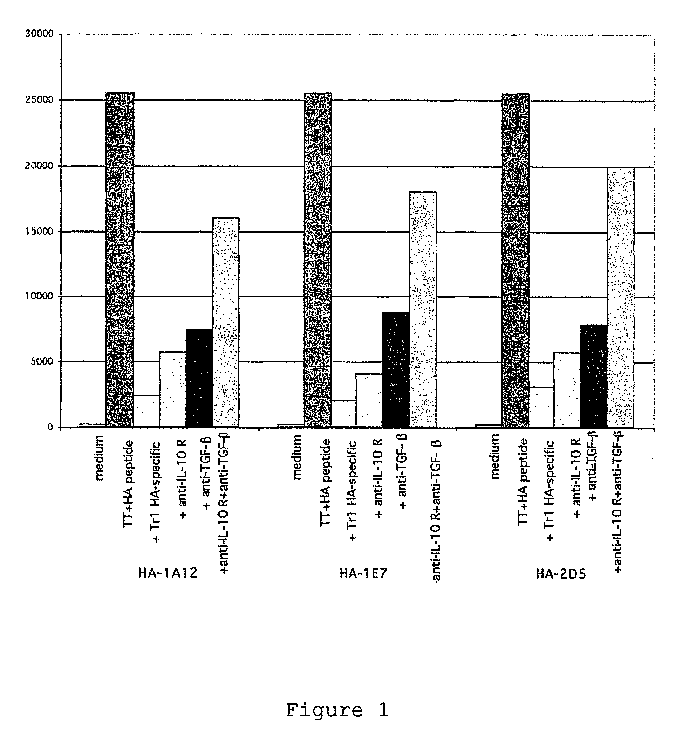

Les résultats obtenus pour 3 clones Tr1 HA spécifiques sont illustrés par la figure 1. Légende de la figure 1 :
En ordonnée : prolifération (thymidine tritiée incorporée, en cpm).
En abscisse :
Medium : cellules T CD4 + non-stimulées ;
TT+ HA peptide : cellules T CD4+ stimulées par le peptide HA 307-319 et l'anatoxine tétanique ;
+Tr1 HA specific : cellules T CD4+ stimulées par le peptide HA 307-319 et l'anatoxine tétanique, en présence des cellules Tr1 spécifiques de l'antigène HA ;
+anti IL-10R : cellules T CD4+ stimulées par le peptide HA 307-319 et l'anatoxine tétanique, en présence des cellules Tr1 spécifiques de l'antigène HA, et d'anticorps anti-IL-10R ;
+anti-TGF-β : cellules T CD4+ stimulées par le peptide HA 307-319 et l'anatoxine tétanique, en présence des cellules Tr1 spécifiques de l'antigène HA, et d'anticorps anti-TGF-β ; +anti IL-10R+anti-TGF-β ; cellules T CD4+ stimulées par le peptide HA 307-319 et l'anatoxine tétanique, en présence des cellules Tr1 spécifiques de l'antigène HA, et d'un mélange d'anticorps anti-IL-10R et d'anticorps anti-TGF-β.

Ces résultats montrent que les lymphocytes Tr1 obtenus par le procédé conforme à l'invention diminuent considérablement la prolifération antigène spécifique des cellules T CD4+. Cet effet inhibiteur n'est que très faiblement atténué par des anticorps anti-IL-10R ou anti-TGF-β séparés, et est partiellement annulé par un mélange des 2 anticorps.

## Revendications

1. Utilisation de cellules présentatrices de l'antigène artificielles, exprimant une molécule du système HLA de classe II et une molécule de LFA-3 humain et n'exprimant aucune des molécules de co-stimulation B7-1, B7-2, B7-H1, CD40, CD23 ou ICAM-1, pour l'obtention de lymphocytes régulateurs Tr1 spécifiques d'antigène.

2. Procédé de préparation de lymphocytes régulateurs Tr1 spécifiques d'antigène à partir des lymphocytes d'un patient, **caractérisé en ce qu'**il comprend :
- l'activation *in vitro* desdits lymphocytes en présence de l'antigène choisi, présenté par des cellules présentatrices de l'antigène artificielles telles que définies dans la revendication 1 ;
- la récupération, à partir desdits lymphocytes, d'une population de lymphocytes T CD4+ activés comprenant au moins 10% de lymphocytes Tr1 spécifiques de l'antigène choisi.

3. Procédé selon la revendication 2, **caractérisé en ce que** les cellules présentatrices de l'antigène artificielles utilisées sont obtenues par co-transfection de cellules de mammifères choisies parmi les fibroblastes, les kératinocytes, les cellules de tubules de rein, les cellules de Schwann, les myoblastes, les cellules endothéliales, avec une séquence d'acide nucléique codant pour la chaîne α d'une molécule HLA de classe II, une séquence d'acide nucléique codant pour la chaîne β d'une molécule HLA de classe II et une séquence d'acide nucléique codant pour le LFA-3 humain.

4. Procédé selon une quelconque des revendications 2 ou 3, **caractérisé en ce que** l'activation *in vitro* desdits lymphocytes s'effectue par co-culture desdits lymphocytes et desdites cellules présentatrices de l'antigène en présence de l'antigène choisi.

5. Procédé selon une quelconque des revendications 2 à 4, **caractérisé en ce qu'**il comprend la répétition de l'étape d'activation *in vitro* des lymphocytes T CD4+ en présence de l'antigène choisi, et la récupération d'une population cellulaire comprenant au moins 30% de lymphocytes Tr1 spécifiques dudit antigène.

6. Procédé selon une quelconque des revendications 2 à 5, **caractérisé en ce qu'**il comprend en outre l'isolement et l'expansion de clones de lymphocytes Tr1 à partir de la population cellulaire récupérée.

7. Procédé de préparation d'une composition pharmaceutique destinée au traitement de maladies inflammatoires et/ou de maladies autoimmunes, **caractérisé en ce qu'**il comprend la préparation de lymphocytes Tr1 spécifiques d'antigène par un procédé selon une quelconque des revendications 2 à 6, et le conditionnement desdits lymphocytes Tr1 sous une formulation appropriée à leur administration à un patient.

## Claims

1. The use of artificial antigen-presenting cells which express a class II molecule of the HLA system and a human LFA-3 molecule and which do not express any of the costimulatory molecules B7-1, B7-2, B7-H1, CD40, CD23 or ICAM-1, for obtaining antigen-specific regulatory Tr1 lymphocytes.

2. A method for the preparation of antigen-specific regulatory Tr1 lymphocytes from the lymphocytes of a patient, **characterized in that** it comprises:
- the *in vitro* activation of said lymphocytes in the presence of the selected antigen, which is presented by artificial antigen-presenting cells as defined in claim 1, and
- recovering from said lymphocytes a population of activated CD4⁺ T lymphocytes comprising at least 10% of Tr1 lymphocytes which are specific for said antigen.

3. The method as claimed in claim 2, **characterized in that** the artificial antigen-presenting cells used are obtained by cotransfection of mammalian cells selected from among fibroblasts, keratinocytes, kidney tubule cells, Schwann cells, myoblasts, endothelial cells, with a nucleic acid sequence encoding the α chain of a class II HLA molecule, a nucleic acid sequence encoding the β chain of a class II HLA molecule and a nucleic acid sequence encoding human LFA-3.

4. The method as claimed in claim 2 or 3, **characterized in that** the *in vitro* activation of said lymphocytes is effected by coculturing said lymphocytes and said antigen-presenting cells in the presence of the antigen selected.

5. The method as claimed in any one of claims 2 to 4, **characterized in that** it comprises the repetition of the step in which CD4⁺ T lymphocytes are activated in vitro in the presence of the antigen selected, and the recovery of a cell population comprising at least 30% of Tr1 lymphocytes which are specific for said antigen.

6. The method as claimed in any one of claims 2 to 5, **characterized in that** it furthermore comprises the isolation and propagation of Tr1 lymphocyte clones from the cell population recovered.

7. The process for the preparation of a pharmaceutical composition intended for the treatment of inflammatory diseases and/or autoimmune diseases, **characterized in that** it comprises preparing antigen-specific Tr1 lymphocytes by a method according to any one of claims 2 to 6 and packaging said Tr1 lymphocytes in a formulation which is suitable for their administration to a patient.

## Patentansprüche

1. Verwendung von künstlichen Antigen-präsentierende Zellen, die ein Molekül des HLA-Klasse-II-Systems und ein menschliches LFA-3-Molekül und keines der costimulatorischen Moleküle B7-1, B7-2, B7-H1, CDE40, CD23 oder ICAM-1 exprimieren, um Antigen-spezifische regulatorische Tr1-Lymphozyten zu erhalten.

2. Verfahren zur Herstellung von Antigen-spezifische regulatorischen Tr1-Lymphozyten aus den Lymphozyten eines Patienten, **dadurch gekennzeichnet, dass** es umfasst:
- die *in vitro* Aktivierung der Lymphozyten in Gegenwart des gewählten Antigens, das von künstlichen Antigen-präsentierenden Zellen nach Anspruch 1 präsentiert wird;
- die Gewinnung aus den Lymphozyten einer Population von aktivierten T CD4+ Lymphozyten, die mindestens 10 % auf das gewählte Antigen-spezifische Tr1-Lymphozyten umfassen.

3. Verfahren nach Anspruch 2, **dadurch gekennzeichnet, dass** die verwendeten künstlichen Antigen-präsentierenden Zellen durch Cotransfektion von Säugerzellen, die aus Fibroblasten, Keratinozyten, Nierentubuluszellen, Schwannzellen, Myoblasten, Endothelzellen gewählt sind, mit einer Nucleinsäuresequenz, die die α-Kette eines Klasse-II-HLA-Moleküls codiert, einer Nucleinsäuresequenz, die die β-Kette eines Klasse-II-HLA-Moleküls codiert, und mit einer Nucleinsäuresequenz, die menschliches LFA-3 codiert.

4. Verfahren nach einem der Ansprüche 2 oder 3, **dadurch gekennzeichnet, dass** die *in vitro* Aktivierung der Lymphozyten durch Cokultur der Lymphozyten und der Antigen-präsentierenden Zellen in Gegenwart des gewählten Antigens erfolgt.

5. Verfahren nach einem der Ansprüche 2 bis 4, **dadurch gekennzeichnet, dass** es die Wiederholung des Aktivierungsschrittes *in vitro* der CD4+ T Lymphozyten in Gegenwart des gewählten Antigens und die Gewinnung einer Zellpopulation, die mindestens 30 % auf das Antigen-spezifische Tr1-Lymphozyten umfasst.

6. Verfahren nach einem der Ansprüche 2 bis 5, **dadurch gekennzeichnet, dass** es ferner die Isolierung und Expansion von Klonen von Tr1-Lymphozyten aus der gewonnenen Zellpopulation umfasst.

7. Verfahren zur Herstellung einer pharmazeutischen Zusammensetzung, die zur Behandlung von entzündlichen und/oder autoimmunen Erkrankungen bestimmt ist, **dadurch gekennzeichnet, dass** es die Herstellung von Antigen-spezifische Tr1-Lymphozyten durch ein Verfahren nach einem der Ansprüche 2 bis 6 und die Konditionierung der Trl-Lymphozyten zu einer Formulierung, die zu ihrer Verabreichung an einen Patienten geeignet ist, umfasst.
